# EUROPEAN PATENT SPECIFICATION

(11) **EP 1 922 984 B1**
(45) Date of publication and mention of the grant of the patent: **01.04.2015**
(21) Application number: 06797720.7
(22) Date of filing: 08.09.2006
(51) Int. Cl.: A61B 1/00, A61B 5/07, G06T 1/00, G09G 5/00, G09G 5/36

(54) **Image display apparatus**
Bildanzeigevorrichtung
Dispositiv d'affichage d'image

(30) Priority: 09.09.2005 JP 2005263109
(43) Date of publication of application: 21.05.2008
(73) Proprietor: OLYMPUS MEDICAL SYSTEMS CORP., Tokyo 151-0072 (JP)
(72) Inventor: KIMOTO, Seiichiro c/o Olympus Medical Systems Corp., Tokyo, 151-0072 (JP)
(74) Representative: von Hellfeld, Axel
(86) International application number: PCT/JP2006/317866
(87) International publication number: WO 2007/029816

(56) References cited:
- EP-A1- 1 922 977
- EP-A2- 0 801 343
- EP-A2- 0 810 794
- JP-A- 2002 176 613
- JP-A- 2004 337 596
- JP-A- 2006 055 262
- US-A- 5 697 885
- US-A1- 2003 228 133

## Description

### TECHNICAL FIELD

The present invention relates to an image display apparatus that displays a series of intra-subject images picked up at time series.

### BACKGROUND ART

In recent years, a swallowable capsule endoscope including an imaging function and a radio communication function has been proposed in the field of endoscopes, and development of an in-vivo information acquiring system for acquiring intra-subject images using the capsule endoscope is underway. The capsule endoscope functions to move in body cavities, e.g., interiors of organs such as the stomach and the small intestine according to peristaltic movements of the organs, and to pick up intra-subject images at intervals of, for example, 0.5 second after being swallowed from a subject's mouth for observation (examination) until being naturally discharged.

During movement of the capsule endoscope in the body of a subject, images picked up by the capsule endoscope are sequentially transmitted to an external receiving apparatus by radio communication. The receiving apparatus includes a radio communication function and a memory function, and sequentially stores the images received from the capsule endoscope in the body of the subject in a memory. By carrying the receiving apparatus, the subject can freely act since swallowing the capsule endoscope until the capsule endoscope is naturally discharged. After the capsule endoscope is naturally discharged from the subject, a doctor or a nurse can diagnose the subject by loading the images accumulated in the memory of the receiving apparatus into an image display apparatus and displaying the images of the organs in the body of the subject on the image display apparatus (see, for example, Patent Document 1).

Patent Document 1: Japanese Patent Application Laid-Open No. 2003-19111

Document EP 0 810 794 A2 concerns a video editing scheme using icons directly obtained from coded video data.
Document US 2003/0228133 A1 concerns a recording apparatus producing a representative image signal of a moving image signal recorded in a recording medium by using a portion of the moving image signal not subjected to an effect process, and discloses an appartaus according to the preamble of claim 1.

### DISCLOSURE OF INVENTION

### PROBLEM TO BE SOLVED BY THE INVENTION

Meanwhile, the capsule endoscope inserted into the body of the subject generally moves in the body of the subject for as long as about eight hours or more, and picks up intra-subject images at intervals of, for example, 0.5 second. Due to this, the number of series of intra-subject images displayed on the image display apparatus is considerably large, and it takes lots of time to observe all the series of intra-subject images. Namely, an occupation period in which one doctor or nurse occupies the image display apparatus to examine and diagnose the subject once is long. Therefore, demand for an image display apparatus that can output moving image data that enable a desired image group partially included in all the intra-subject images, i.e., an image group which the doctor or nurse desires to observe, to be played as a moving image rises.

The present invention has been achieved under these circumstances, and it is an object of the present invention to provide an image display apparatus that can output moving image data that enables a desired image group partially included in all intra-subject images to be played as an moving image.

### MEANS FOR SOLVING PROBLEM

Some or all of the above problems are overcome by an image display apparatus having the features of claim 1.

In the image display apparatus according to the invention, the input unit may input the image designation information for designating a plurality of central images from among the series of images, the image extracting unit may extract, from the series of images, a plurality of partial image groups in which the plurality of central images designated by the image designation information are arranged at the respective centers, and the image generator may generate a combined moving image obtained by combining the plurality of partial image groups along time series, based on the plurality of partial image groups extracted by the image extracting unit.

In the image display apparatus according to the invention, the input unit may further input end-image designation information for designating two end images from among the series of images, the image extracting unit may extract, from the series of images, a partial continuous image group in which the two end images designated by the end-image designation information are arranged on both ends, respectively, and the image generator may generate a clipped moving image including the partial continuous image group, based on the partial continuous image group extracted by the image extracting unit.

In the image display apparatus according to the invention, the input unit may further input mode designation information for designating an output mode of a moving image generated by the image generator as one of the partial moving image, the combined moving image, and the clipped moving image, and the image generator may generate the moving image in the output mode designated by the mode designation information.

### EFFECT OF THE INVENTION

According to the present invention, it is advantageously possible to realize the image display apparatus that can generate and output moving image data that enables desired images included in all images of a desired subject, e.g., a partial image group including images of a region of interest such as a bleeding site or an affected region, to be played as an moving image.

### BRIEF DESCRIPTION OF DRAWINGS

FIG. 1 is a pattern diagram typically showing an example of a configuration of an in-vivo information acquiring system including an image display apparatus according to an embodiment of the present invention.
FIG. 2 is a block diagram typically showing an example of a configuration of the image display apparatus according to the embodiment of the present invention.
FIG. 3 is a pattern diagram typically showing a specific example of a display screen of a display unit.
FIG. 4 is a pattern diagram showing an example of a state in which a window for setting an output mode of an image file is displayed.
FIG. 5 is a pattern diagram for explaining an operation for creating a normal moving-image file.
FIG. 6 is a pattern diagram for explaining an operation for creating a combined moving-image file.
FIG. 7 is a pattern diagram for explaining an operation for creating a clipped moving-image file.
FIG. 8 is a pattern diagram showing a specific example of a window displayed on the display unit for performing an image-file output operation or the like.
FIG. 9 is a pattern diagram showing a specific example of a window displayed on the display unit for creating a report on a subject.
FIG. 10 is a pattern diagram showing a specific example of a window for replacing an order of created groups.
FIG. 11 is a pattern diagram showing an example of a window in which a group processor has set grouped images.
FIG. 12 is a pattern diagram showing a specific example of a report created by a report creator.
FIG. 13 is a pattern diagram for explaining a data lock processing performed on an original file by a lock processor.

### EXPLANATIONS OF LETTERS OR NUMERALS

- 1: Subject
- 2: Capsule endoscope
- 3: Receiving apparatus
- 3a-3h: Receiving antenna
- 4: Image display apparatus
- 11: Input unit
- 12: Display unit
- 13: Card I/F
- 14: Information input/output I/F
- 15: Storage unit
- 15a: Examination folder
- 15b: Management folder
- 15c: Input/output folder
- 16: Control unit
- 16a: Display controller
- 16b: Image file creator
- 16c: Report creator
- 16d: Group processor
- 16e: Image extracting unit
- 16f: Output processor
- 16g: Lock processor
- 16h: Input processor
- 50: Main-image display area
- 60: File display area
- 70: Display-operation icon group
- 80: Subimage display area
- 81: Scrollbar
- 91: Image output icon
- 92: Report icon
- 93-95: Number-of-displayed-images setting icon
- 96: Exit icon
- 101: Still-image file display area
- 102: Moving-image file display area
- 103: Output-file display area
- 104: File designation icon
- 105: Remove icon
- 110: Display-operation icon group
- 120: Output-destination selection area
- 130: OK icon
- 201: Thumbnail display area
- 202: Scrollbar
- 203: Grouped-image display area
- 204: Scrollbar
- 210: Normal-image display area
- 211-213: Marker icon
- 214: Comment creating area
- 215: Dictionary area
- 216: Entry area
- 221: Undo icon
- 222: Grouping icon
- 223: Group arrangement icon
- 224: Report creation icon
- 225: Close icon
- 250: Grouped area
- 251: Scrollbar
- 261: OK icon
- 262: Cancel icon
- 301: Patient information
- 302: Examination information
- 303: Diagnosis information
- 304: Examination result information
- 400: External computer
- 401: Portable recording medium
- A1-A5, D1, D2, B1-B5, C1-C5, E1-E5: Image
- CPG: Partial image group
- CF: Copy file
- F1-F4: Examination file
- Gr1-Gr4: Grouped image
- K: Cursor
- M1, M2: Normal moving image
- MA: Combined moving image
- MB: Clipped moving image
- OF: Original file
- P1: Image
- PG: Image group
- PL1-PL4: Imaged region marker
- PV: Preview area
- RM1-RM5: Report marker
- SP1-SP5: Thumbnail image
- S: Slider
- TS: Timescale
- W1-W6: Window

### BEST MODE(S) FOR CARRYING OUT THE INVENTION

Exemplary embodiments of an image display apparatus according to the present invention will be described hereinafter. It is to be noted that the present invention is not limited by the embodiments.

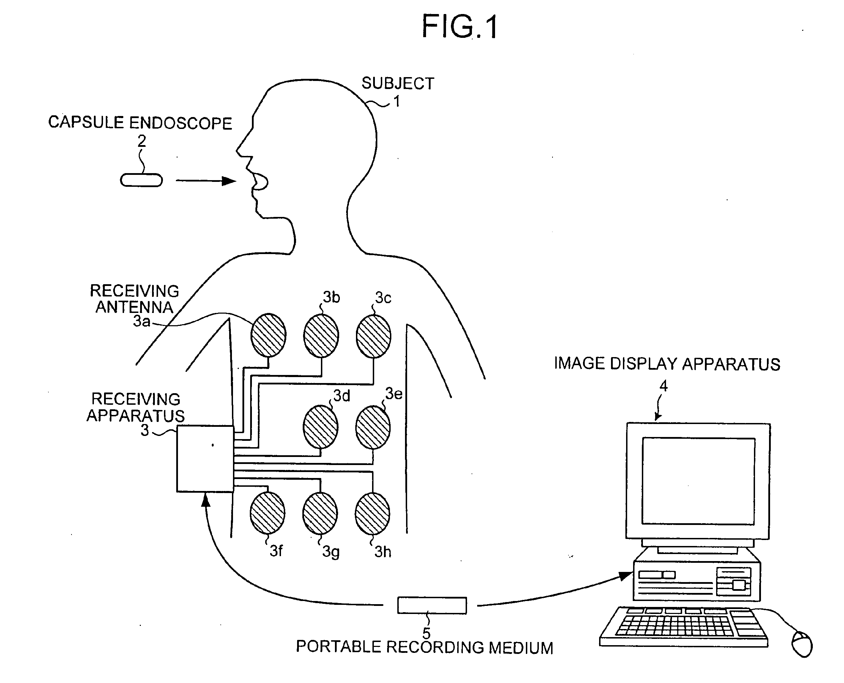

FIG. 1 is a pattern diagram typically showing an example of a configuration of an in-vivo information acquiring system including the image display apparatus according to an embodiment of the present invention. As shown in FIG. 1, the in-vivo information acquiring system includes a capsule endoscope 2 that moves along a passing route in a body of a subject 1 and that picks up images on an interior of the subject 1, a receiving apparatus 3 that receives radio signals transmitted by the capsule endoscope 2 and that accumulates images included in the received radio signals, an image display apparatus 4 that displays the images accumulated in the receiving apparatus, i.e., the images picked up by the capsule endoscope 2, and a portable recording medium 5 that mediates between the receiving apparatus 3 and the image display apparatus 4 for transmitting and receiving information therebetween.

The capsule endoscope 2, which is inserted into the interior of the subject 1, includes an imaging function that sequentially picks up the images of the interior of the subject 1 at time series, and a radio communication function that transmits the radio signals including the picked-up images to the outside. The capsule endoscope 2 passes through the esophagus in the body of the subject 1 by being swallowed by the subject 1, and moves in body cavities according to a peristaltic movement of a digestive canal. At the same time, the capsule endoscope 2 sequentially picks up the images of the interior of the subject 1 at predetermined intervals, e.g., intervals of 0.5 seconds, and sequentially transmits the images of the interior of the subject 1 to the receiving apparatus 3 through a predetermined radio wave.

The receiving apparatus 3, to which a plurality of antennas 3a to 3h arranged to be distributed on, for example, the body surface of the subject 1 are connected, receives the radio signals from the capsule endoscope 2 through one of the receiving antennas 3a to 3h, and acquires the images of the interior of the subject 1 based on the received radio signals. Furthermore, the receiving apparatus 3, to which the portable recording medium 5 is detachably inserted, sequentially stores the images sequentially acquired based on the radio signals from the capsule endoscope, i.e., the images picked up by the capsule endoscope 2 in the portable recording medium 5. In this manner, the receiving apparatus 3 accumulates an image group of the images of the interior of the subject 1 picked up by the capsule endoscope 2 at time series in the portable recording medium 5.

Each of the receiving antennas 3a to 3h, which is realized by, for example, a loop antenna, receives the radio signal transmitted by the capsule endoscope 2. As shown in FIG. 1, the receiving antennas 3a to 3h are arranged to be distributed to predetermined positions on the body surface of the subject 1, e.g., at positions corresponding to the passing route of the capsule endoscope 2 in the subject, respectively. Alternatively, the receiving antennas 3a to 3h can be arranged to be distributed to predetermined positions on a jacket which the subject 1 is to wear, respectively. In this alternative, by causing the subject 1 to wear the jacket, the receiving antennas 3a to 3h are arranged to be distributed to the predetermined positions on the body surface of the subject 1 corresponding to the passing route of the capsule endoscope 2 in the subject 1, respectively. It suffices to arrange one or more receiving antennas for the subject 1, and the number of arranged receiving antennas is not particularly limited to eight.

The portable recording medium 5 is a portable recording media such as a compact flash (registered trademark). The portable recording medium 5 has a structure in which the portable recording medium 5 is detachable from the receiving apparatus 3 or the image display apparatus 4, and can output and record information when being attached to the receiving apparatus 3 or the image display apparatus 4. Specifically, when being attached into the receiving apparatus 3, the portable recording medium 5 sequentially stores therein various information such as the images picked up by the capsule endoscope 2 and acquired by the receiving apparatus 3 and imaging time of each of the images. When being attached into the image display apparatus 4, the portable recording medium 5 outputs stored information such as the images picked up by the capsule endoscope 2 to the image display apparatus 4. In this manner, the information stored in the portable recording medium 5 is loaded into the image display apparatus 4. Moreover, information on the subject 1 for a capsule endoscopic examination and the like are written to the portable recording medium 5 by the image display apparatus 4. The capsule endoscopic examination means observing the images picked up by the capsule endoscope 2 by inserting the capsule endoscope 2 into the interior of the subject 1.

The image display apparatus 4 is to display the images picked up by the capsule endoscope 2 or the like. Specifically, the image display apparatus 4 is configured, like a workstation or the like, to acquire various information such as the images picked up by the capsule endoscope 2 by causing the receiving apparatus 3 to load the various information accumulated in the portable recording medium 5 into the image display apparatus 4, and to display the images of the interior of the subject 1 based on the acquired information. Such an image display apparatus 4 includes an image display function of sequentially displaying the respective images included in the image group of the images of the interior of the subject 1, and a processing function of causing a user such as a doctor or a nurse to observe (examine) the images of the interior of the subject 1 and to diagnose the subject 1. In this case, the user can observe (examine) regions in the body of the subject 1, e.g., the esophagus, the stomach, the small intestine, and the large intestine by sequentially displaying the images of the interior of the subject 1 on the image display apparatus 4, and diagnose the subject 1 based on the observation (examination).

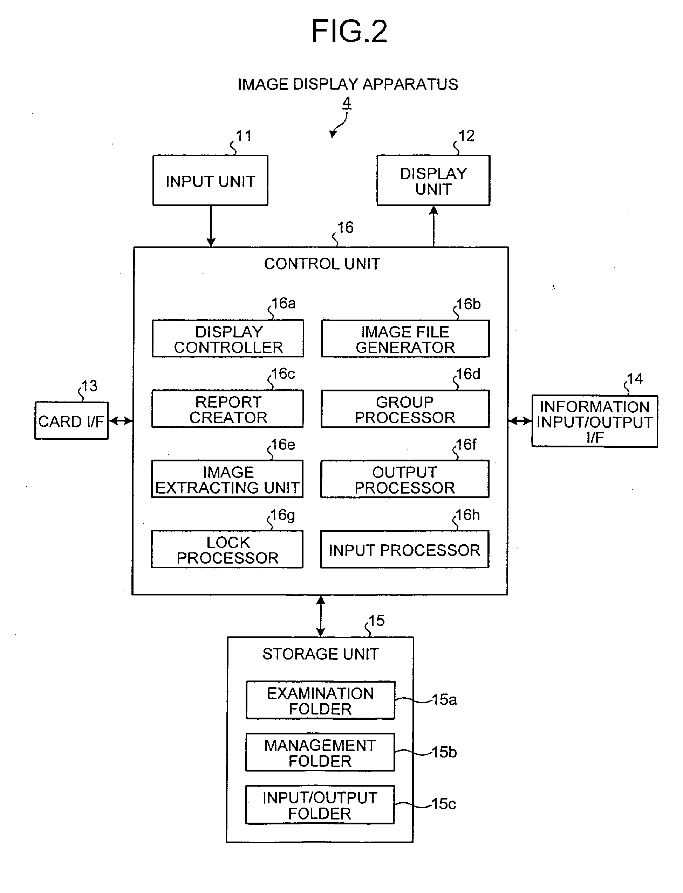

A configuration of the image display apparatus 4 will next be described. FIG. 2 is a block diagram typically showing an example of a configuration of the image display apparatus 4 according to the embodiment of the present invention. As shown in FIG. 2, the image display apparatus 4 includes an input unit 11 that receives various information for observing the images of the interior of the subject 1, a display unit 12 that displays various information such as the images of the interior of the subject 1 for examining and diagnosing the subject 1, and a card interface (I/F) 13 for fetching the information, e.g., the images of the interior of the subject 1, accumulated and stored in the portable recording medium 5. The image display apparatus 4 also includes an information input/output I/F 14 for inputting/outputting the various information such as the images of the interior of the subject 1, for example, from/to an external computer, a storage unit 15 that stores therein the various information such as the images of the interior of the subject 1, and a control unit 16 that controls driving of the respective constituent elements of the image display apparatus 4.

The input unit 11, which is realized by a keyboard, a mouse and the like, inputs instruction information on an instruction to the control unit 16 and patient information on the subject 1 to the control unit 16 according to a user's input operation. It is to be noted that the patient information is registered in the receiving apparatus 3, for example, through the portable recording medium 5 so as to initialize the receiving apparatus 3 as a receiving apparatus for carrying out a capsule endoscopic examination on the subject 1. Examples of the patient information include a patient name, a sex, a birth date, and a patient ID of the subject 1.

The display unit 12, which is realized by using a display of various types such as a CRT display or a liquid crystal display, displays various information instructed to be displayed by the control unit 16. In this case, the display unit 12 displays the various information such as the images of the subject 1 picked up by the capsule endoscope 2 so as to observe and diagnose the subject 1. A specific example of a display screen of the display unit 12 will be described later.

The card I/F 13 is to fetch the information stored in the portable recording medium 5. Specifically, the card I/F 13, into which the portable recording medium 5 is detachably attached, reads the information accumulated and stored in the portable recording medium 5, and transfers the stored information thus obtained to the control unit 16. Furthermore, the card I/F 13 writes information instructed to be written by the control unit 16, e.g., the patient information to the portable recording medium 5 attached into the card I/F 13.

The information input/output I/F 14 is to input/output various information between, for example, the external computer or a peripheral device and the image display apparatus 4. Specifically, the information input/output I/F 14, into which the portable recording medium such as a flexible disk (FD), a compact disk (CD) or a DVD (Digital Versatile Disk) is detachably attached, is realized using a drive or the like that performs a various-information read processing or write processing on the portable recording medium attached therein. The information input/output I/F 14 has a structure connectable to the peripheral device such as a printer through a predetermined cable. Such an information input/output I/F 14 writes the information instructed to be written by the control unit 16 to the portable recording medium in the portable recording medium in the drive, or outputs the information instructed to be output by the control unit 16 to the peripheral device such as the printer. Moreover, the information input/output I/F 14 reads information instructed to be read by the control unit 16 from the portable recording medium in the drive, and transfers the obtained information to the control unit 16.

The storage unit 15, which is realized by an information recording unit such as a RAM, an EEPROM or a hard disk capable of accumulating and reading information, stores therein the information instructed to be written by the control unit 16, and transmits the stored information instructed to be read by the control unit 16 to the control unit 16. Such a storage unit 15 includes an examination folder 15a for holding and managing a subject examination file for each subject including the image group of the images of the subject, the patient information, and examination information (e.g., an examination date and an examination ID), a management folder 15b for holding and managing various files of a clinical chart (report) for each subject created by a processing function of the image display apparatus 4, still images, and moving images, and an input/output folder 15c for holding and managing various files to be input/output to/from the outside.

As described, the control unit 16 controls driving of the respective constituent elements of the image display apparatus 4, e.g., the input unit 11, the display unit 12, the card I/F 13, the information input/output I/F 14, and the storage unit 15, and controls input/output of information among the respective constituent elements. The control unit 16 creates an examination file obtained by making the image group of the images of the subject 1 acquired through the portable recording medium 5, time information on imaging time of each image included in the image group, the patient information on the subject 1, and the examination information on the subject 1 correspond to one another into a file, and stores the acquired examination file in the examination folder 15a. In this case, the control unit 16 holds and manages each examination file stored in the examination folder 15a per, for example, subject or examination ID.

Moreover, the control unit 16 includes a display controller 16a that controls a display operation for displaying various information performed by the display unit 12, an image file creator 16b that creates the image file of desired still images or moving images based on the image group included in the examination file, a report creator 16c that creates a report in which a diagnosis result or the like about each subject is recorded, and a group processor 16d that divides the images recorded in the report created by the report creator 16c into desired groups. The control unit 16 also includes an image extracting unit 16e, which extracts images necessary to create the image file or report from among those in the image group included in the examination file, an output processor 16f, which performs an output processing for the data of the image file, the report file or the like to be output to the outside, a lock processor 16g that prohibits change in an original file a copy file of which is output to the outside, and an input processor 16h that performs an input processing for the data of the image file, the report file or the like input from the outside.

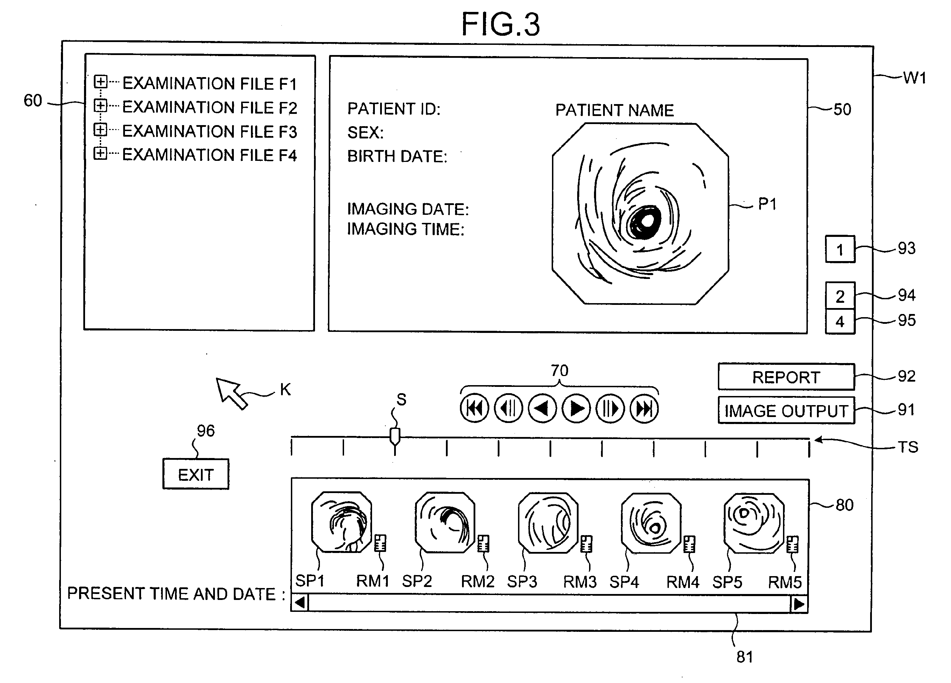

An operation performed by the display controller 16a that controls the display operation performed by the display unit 12 will next be described while specifically showing examples of a display screen of the display unit 12. FIG. 3 is a pattern diagram typically showing a specific example of the display screen of the display unit 12. The display controller 16a controls the display unit 12 to display a window W1 shown in FIG. 3 if the control unit 16 performs a predetermined login processing.

As shown in FIG. 3, a main-image display area 50 for displaying the images picked up by the capsule endoscope 2, a file display area 60 for displaying a list of examination files stored in the examination folder 15a of the storage unit 15, a display-operation icon group 70 for performing an image display operation for displaying each image in the image group included in each of the examination files in the main-image display area 50, a subimage display area 80 for displaying thumbnails corresponding to the images selected from among those sequentially displayed in the main-image display area 50, and a scrollbar 81 for scrolling each of the thumbnails displayed in the subimage display area 80 are formed in the window W1.

Moreover, a timescale TS indicating passing time since start of picking up each image included in the examination file selected from among those in the file display area 60, and a slider S indicating the passing time of the image displayed in the main-image display area 50 among the passing time indicated by the timescale TS are formed in the window W1. Moreover, an mage output icon 91 for outputting the image file created by the image file creator 16b, a report icon 92 for creating the report on each subject, number-of-displayed-images setting icons 93 to 95 for setting the number of images to be displayed simultaneously in the main-image display area 50, and an Exit icon 96 for closing the window W1 are formed in the window W1.

Furthermore, a cursor K for performing designation of one examination file, image selection, operation of the various icons, and the like by an input operation using the input unit 11, and information indicating current date and time are displayed in the window W1.

The display controller 16a displays the image group included in the examination file designated by the input operation using the input unit 11 from among, for example, examination files F1 to F4 in the list displayed in the file display area 60, in the main-image display area 50. Specifically, the user performs the input operation for designating a desired examination file from among the examination files F1 to F4 by putting the cursor K on the desired examination file using the input unit 11. The display controller 16a sequentially displays images P1 in the image group included in the designated examination file in the main-image display area 50. In this case, the display controller 16a sequentially displays the images P1 in, for example, a forward or backward direction of the time series to correspond to the display operation icon designated by the input operation using the input unit 11 from among those in the display-operation icon group 70. Furthermore, the display controller 16a sequentially displays the patient information (the patient name, the sex, the birth date, and the patient ID), an imaging date, and imaging time of the images P1 in the main-image display area 50 according to the images P1 thus sequentially displayed.

The display controller 16a displays the images P1 in the main-image display area 50 one by one if an input operation for putting the cursor K on and designating the number-of-displayed-images setting icon 93 from among the number-of-displayed-images setting icons 93 to 95 is performed using the input unit 11, and displays two images P1 in the main-image display area 50 simultaneously if an input operation for putting the cursor K on and designating the number-of-displayed-images setting icon 94 is performed using the input unit 11, and displays four images P1 in the main-image display area 50 simultaneously if an input operation for putting the cursor K on and designating the number-of-displayed-images setting icon 95 is performed using the input unit 11.

Moreover, the display controller 16a moves the slider S in the forward or backward direction of the time series along the timescale TS synchronously with switchover of the images sequentially displayed in the main-image display area 50. In this case, the slider S moves so as to point out a position of the image that is being displayed in the main-image display area 50 on the timescale TS on which the passing time since, for example, the start of imaging is put.

Furthermore, if the input operation for putting the cursor K on a desired image and thereby selecting and designating the desired image from among the images sequentially displayed in the main-image display area 50 is performed using the input unit 11, the display controller 16a displays a thumbnail corresponding to the selected and designated image in the subimage display area 80. Specifically, the input unit 11 inputs image designation information for selecting and designating the desired image from among the images displayed in the main-image display area 50, to the control unit 16. Whenever the image designation information for selecting and designating the desired image is input to the control unit 16 by the input unit 11, the display controller 16a sequentially adds the thumbnail corresponding to the image selected and designated by the image designation information. In this case, as shown in, for example, FIG. 3, the display controller 16a displays thumbnails SP1 to SP5 in the subimage display area 80. If an input operation for selecting and designating, for example, the thumbnail SP1 from among those displayed in the subimage display area 80 is performed, the display controller 16a displays the image corresponding to the selected, designated thumbnail SP1 in the main-image display area 50.

If a report in which the thumbnail displayed in the subimage display area 80 is recorded is created, the display controller 16a displays a report marker indicating that the report has been created near the thumbnail. In this case, if reports are created for, for example, the thumbnails SP1 to SP5, the display controller 16a displays report markers RM1 to RM5 near the thumbnails SP1 to SP5, respectively as shown in FIG. 3. If an input operation is performed for selecting and designating a desired report marker from among those displayed near the respective thumbnails by putting the cursor on the desired report marker, the display controller 16a displays the report corresponding to the selected report marker so that the user can view the report.

Next, an operation performed by the image file creator 16b for creating an image file of still images or moving images based on the image group included in each examination file will be described. The image file creator 16b functions as image creator that generates still images or moving images of the subject, and creates an image file based on the image group included in the desired examination file selected from among those stored in the examination folder 15a. In this case, the image file creator 16b creates an image file in an output mode designated by designation information input from the input unit 11 to the control unit 16.

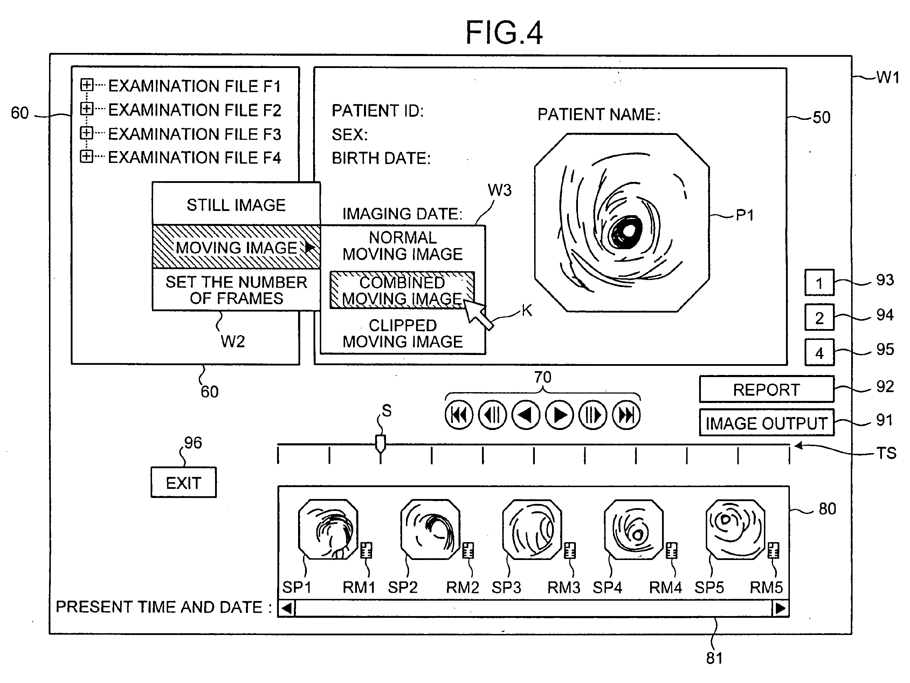

FIG. 4 is a pattern diagram showing an example of a state in which a window for setting the output mode of the image file is displayed. The user displays a window W2 shown in FIG. 4 by performing an input operation using the input unit 11. In the window W2, a menu for setting the output mode of the image file created by the image file creator 16b is displayed. If designation information for selecting and designating "still image" in the menu shown in the window W2 is input from the input unit 11, the image file creator 16b creates a still image file based on the image group included in the desired examination file.

Specifically, the user performs an input operation for designating a desired thumbnail from among those in the subimage display area 80 by putting the cursor K on the desired thumbnail. In this case, the input unit 11 inputs image designation information for designating the image of the subject corresponding to the desired thumbnail to the control unit 16. The image extracting unit 16e extracts the image designated by the image designation information from those in the image group included in the desired examination file. The image file creator 16b creates the still image file based on the image extracted by the image extracting unit 16e, and stores the created still image file in the management folder 15b of the storage unit 15.

The still image file thus created includes a still image identical in display content to the image (thumbnail) designated by the image designation information input from the input unit 11, and is in an output mode in which a still image can be output in a general-purpose still-image data format such as GIF, JPEG or TIF.

If designation information for selecting and designating "moving image" in the menu shown in the window W2 shown in FIG. 4 is input from the input unit 11, the image file creator 16b creates one of a normal moving-image file, a combined moving-image file, and a clipped moving-image file based on the image group included in the desired examination file. The normal moving-image file includes a normal moving image obtained by arranging the image designated by the image designation information input from the input unit 11 and a predetermined number of frame images continuous to the designated image at time series. The combined moving-image file includes a combined moving image (Combined Movie) obtained by combining a plurality of normal moving images at time series. The clipped moving-image file includes a clipped moving image (clipped movie) obtained by arranging two images designated by the image designation information input from the input unit 11 and one or more images present between the two images at time series. Each of the normal moving-image file, the combined moving-image file, and the clipped moving-image file is in an output mode in which a moving image can be output in a general-purpose moving image data format such as WMV or MPEG.

Specifically, if the designation information for selecting and designating "moving image" in the menu in the window W2 is input from the input unit 11, a window W3 for setting the output mode of the moving image file created by the image file creator 16b in more detail is displayed as shown in FIG. 4. The user performs an input operation for selecting and designating a desired output mode by putting the cursor K on one of "normal moving image", "combined moving image", and "clipped moving image" in the menu in the window W3, and then performs an input operation for selecting and designating one or more desired thumbnails from among those in the subimage display area 80. In this case, the input unit 11 inputs the designation information for selecting and designating one of "normal moving image", "combined moving image", and "clipped moving image" to the control unit 16. The image file creator 16b creates a moving image file including one of the normal moving image, the combined moving image, and the clipped moving image matched to the item in the window W3 designated by the designation information from the input unit 11 based on one or more images corresponding to one or more desired thumbnails (that is, one or more desired thumbnails selected and designated from those in the subimage area 80).

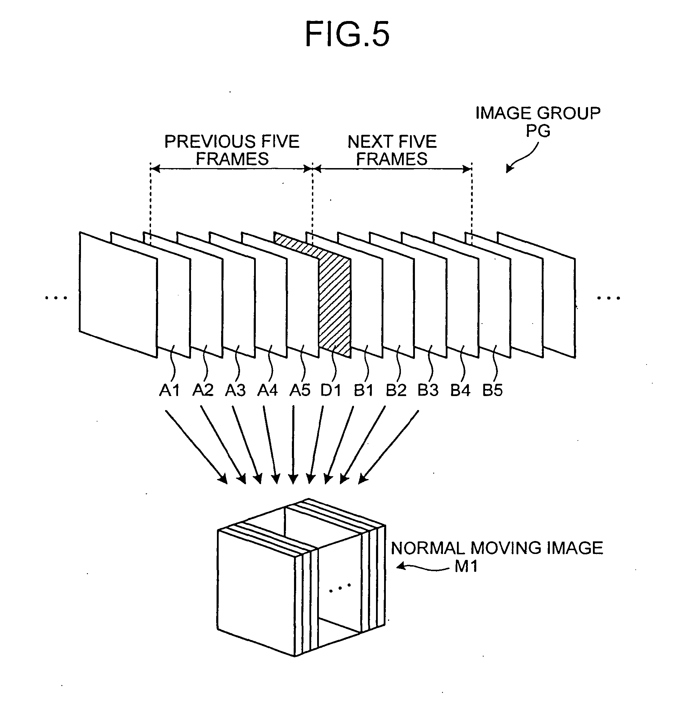

Next, an operation performed by the image file creator 16b for creating a moving image file including the normal image file, that is, a normal moving-image file will be described in more detail. FIG. 5 is a pattern diagram for explaining the operation for creating the normal moving-image file. If designation information for selecting and designating "normal moving image" in the menu shown in the window W3 is input from the input unit 11, and image designation information for selecting and designating the desired thumbnail from among those in the subimage display area 80 is input from the input unit 11, the image extracting unit 16e first extracts images necessary to create a normal moving image from among those in an image group PG included in the desired examination file selected by the user in advance. In this case, the image extracting unit 16e extracts an image D1 corresponding to the desired thumbnail designated by the image designation information from the input unit 11, a predetermined number of continuous frames, e.g., five frame images A1 to A5 previous to the image D1 temporally, and a predetermined number of continuous frames, e.g., five frame images B1 to B5 next to the image D1 temporally.

The image D1 designated by the image designation information is a central image arranged at the center of a partial image group (e.g., an image group including the images A1 to A5, the image D1, and the images B1 to B5) extracted from the image group PG for creating one normal moving image.

The image file creator 16b creates the normal moving-image file based on the images A1 to A5, D1, and B1 to B5 extracted by the image extracting unit 16e. Specifically, the image file creator 16b arranges the images A1 to A5, D1, and B1 to B5 at time series, performs a compression processing on the images A1 to A5, D1, and B1 to B5, and creates a normal moving image M1 that can be displayed by continuously playing the images from the first image A1 to the last image B5. The normal moving image M1 is a partial moving image created based on the partial image group extracted partially from the image group PG included in the desired examination file. The image file creator 16b converts a format of the normal moving image M1 into the general-purpose moving-image data format, and creates the normal moving-image file by making the normal moving image M1 in the form of a file. Thereafter, the control unit 16 stores the obtained normal moving-image file in the management folder 15b of the storage unit 15. A data capacity of the normal moving image M1 included in the normal moving-image file can be made small as compared with a total data capacity of the images A1 to A5, D1, and B1 to B5 for forming the normal moving image M1.

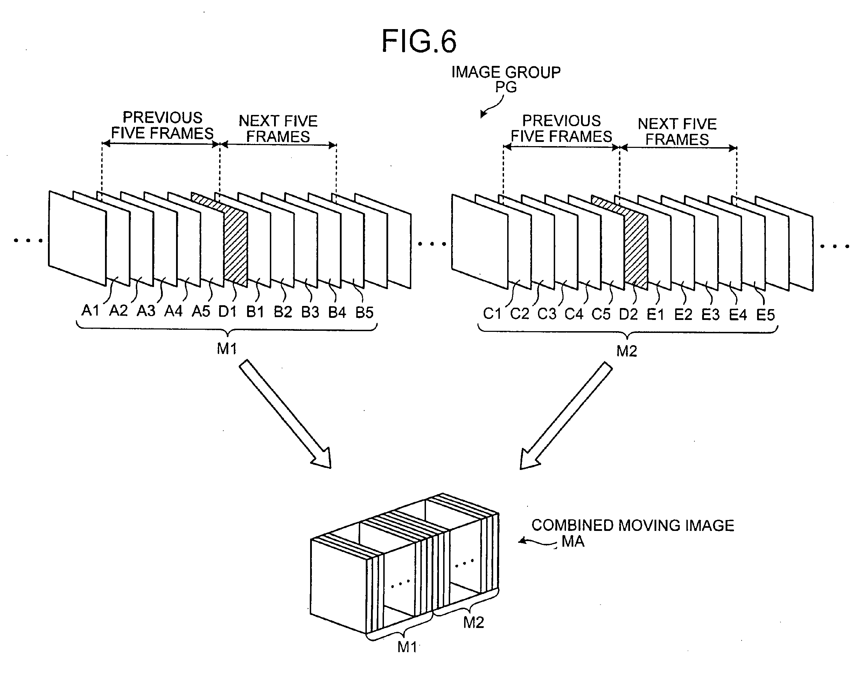

An operation performed by the image file creator 16b for creating a moving image file including the combined moving images, that is, a combined moving-image file will be described in more detail. FIG. 6 is a pattern diagram for explaining the operation for creating the combined moving-image file. If designation information for selecting and designating "combined moving image" in the menu shown in the window W3 is input from the input unit 11, and image designation information for selecting and designating two desired thumbnails from those in the subimage display area 80 is input from the input unit 11, the image extracting unit 16e extracts first images necessary for the processing for creating a combined moving image from among those in the image group PG included in the desired examination file selected by the user in advance as shown in FIG. 6. In this case, the image extracting unit 16e extracts images D1 and D2 corresponding to the desired thumbnail designated by the image designation information from the input unit 11, respectively, a predetermined number of temporally continuous frames, e.g., five frame images A1 to A5 and C1 to C5 previous to the respective images D1 and D2, and a predetermined number of temporally continuous frames, e.g., five frame images B1 to B5 and E1 to E5 next to the respective images D1 and D2.

The two images D1 and D2 designated by the image designation information are central images arranged at centers of two partial images (e.g., an image group including the images A1 to A5, the image D1, and the images B1 to B5 and an image group including the images C1 to C5, the image D2, and the images E1 to E5) extracted from the image group PG, respectively.

As already stated, the image file creator 16b arranges the images A1 to A5, D1, and B1 to B5 extracted by the image extracting unit 16e at time series, performs a compression processing on the images A1 to A5, D1, and B1 to B5, and creates the normal moving image M1. The image file creator 16b arranges the images C1 to C5, D2, and E1 to E5 extracted by the image extracting unit 16e at time series, performs a compression processing on the images C1 to C5, D2, and E1 to E5, and creates a normal moving image M2 that can be displayed by continuously playing the images from the first image C1 to the last image E5 almost similarly to the normal moving image M1. The image file creator 16b performs a compression processing on the last image B5 in the normal moving image M1 and the first image C1 in the normal moving image M2 to combine the compressed images B5 and C1, and creates a combined moving image MA in which the normal moving images M1 and M2 are combined at time series. The image file creator 16b converts a data format of the combined moving image MA into the general-purpose moving-image data format, and creates the combined moving-image file by making the combined moving image MA in the form of a file. Thereafter, the control unit 16 stores the obtained combined moving-image file in the management folder 15b of the storage unit 15.

Alternatively, the image file creator 16b can create the combined moving image MA by arranging the images A1 to A5, D1, and B1 to B5 extracted by the image extracting unit 16e at time series, arranging the image C1 right after the image B5, arranging the images C1 to C5, D2, and E1 to E5 at time series, and performing a compression processing on the images thus arranged at time series.

If the two or more image groups extracted by the image extracting unit 16e for creating the combined moving image include overlapped images, the image file creator 16b deletes one of the overlapped images and creates the combined moving image. For example, if the images B4 and B5 are overlapped with the images C1 and C2 in the images B1 to B5 and C1 to C5, respectively, the image file creator 16b deletes one of the overlapped images B4 and C1 and one of the images B5 and C2 and creates the combined moving image MA.

If image designation information for selecting and designating three or more desired thumbnails from among those in the subimage display area 80 is input from the input unit 11, the image file creator 16b creates a combined moving image using three or more image groups extracted by the image extracting unit 16e, that is, three or more images (central images) corresponding to the three or more thumbnails designated by the image designation information from the input unit 11, respectively, a predetermined number of temporally continuous frame images (previous images) previous to each of the three or more central images, and a predetermined number of temporally continuous frame images (next images) next to each of the three or more central images. In this case, the created combined moving image includes three or more image groups each including the central image, the predetermined number of previous frame images, and the predetermined number of next frame images.

The number of extracted previous images or next images extracted together with each central image, that is, the predetermined number of frames can be set to a desired number of frames if designation information for selecting and designating "number-of-frames setting" in the menu in the window W2 shown in FIG. 5 is input from the input unit 11. Specifically, if the designation information for selecting and designating "number-of-frames setting" in the window W2 is input from the input unit 11, a frame setting screen (not shown) is displayed. If a desired number of frames are input or selected by the input operation using the input unit 11, the control unit 16 sets the number of frames of the previous images or the next images extracted together with each central image to the number of frames input or selected on the frame setting screen. The number of frames thus set can be applied if the normal moving image is to be generated.

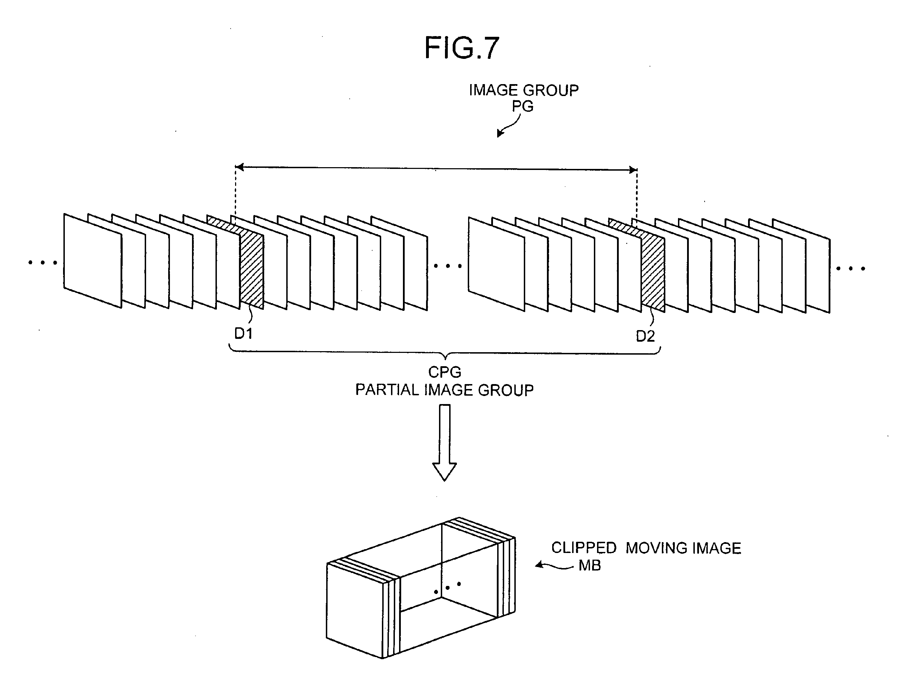

An operation performed by the image file creator 16b for creating a moving image file including the clipped moving images, that is, a clipped moving-image file will be described in more detail. FIG. 7 is a pattern diagram for explaining an operation for creating the clipped moving-image file. If designation information for selecting and designating "clipped moving image" in the menu shown in the window W3 is input from the input unit 11, and image designation information for selecting and designating two desired thumbnails from those in the subimage display area 80 is input from the input unit 11, the image extracting unit 16e extracts images necessary for the processing for creating the clipped moving image from the image group PG included in the desired examination file selected by the user in advance as shown in FIG. 7. In this case, the image extracting unit 16e extracts a partial image group CPG including two images D1 and D2 corresponding to the desired thumbnails designated by the image designation information from the input unit 11, respectively, and all images present between the images D1 and D2.

The two images D1 and D2 thus extracted are end images arranged on both ends of the partial image group CPG, respectively, and arranged at the first or last of the partial image group CPG at time series. Namely, the images included in the partial image group CPG are temporally continuous images from the first image D1 to the last image D2.

The image file creator 16b arranges the respective images included in the partial image group CPG extracted by the image extracting unit 16e at time series, performs a compression processing on the images, and creates a clipped moving image MB that can be displayed by continuously playing the respective images from the first image D1 to the last image D2. The image file creator 16b converts a data format of the clipped moving image MB into the general-purpose moving-image data format, and creates the clipped moving-image file by making the clipped moving image in the form of a file. Thereafter, the control unit 16 stores the obtained clipped moving-image file in the management folder 15b of the storage unit 15. A data capacity of the clipped moving image MB included in the clipped moving-image file can be made smaller than a total data capacity of the respective images in the partial image group CPG that forms the clipped moving image MB.

In this manner, the image file creator 16b can create the image file in the desired output mode designated from among the still images, the normal moving image, the combined moving image, and the clipped moving image. In this case, the image file creator 16b can easily create the still images, the normal moving image, the combined moving image or the clipped moving image for displaying images of the region of interest, e.g., a bleeding site, an affected region or the like of the subject from the image group included in the desired examination file. Moreover, if the image file creator 16b creates a plurality of normal moving images for displaying images of, for example, the bleeding site, the affected region or the like of the subject, these normal moving images can be integrated into one combined moving image. Therefore, the image file creator 16b can realize the moving image file (normal moving-image file, combined moving-image file or clipped moving-image file) for displaying images of the region of interest among the image group of the subject, the data capacity of which can be reduced as much as possible and which is suitably output to, for example, an external computer or suitably subjected to a moving-image play processing.

Each of the image files (the still image file, the normal moving-image file, the combined moving-image file, and the clipped moving-image file) created by the image file creator 16b can be output in the general-purpose data format. Due to this, if the image file is output to, for example, the external computer, the image file can be easily played as still images or a moving image by a general-purpose image display application of the external computer.

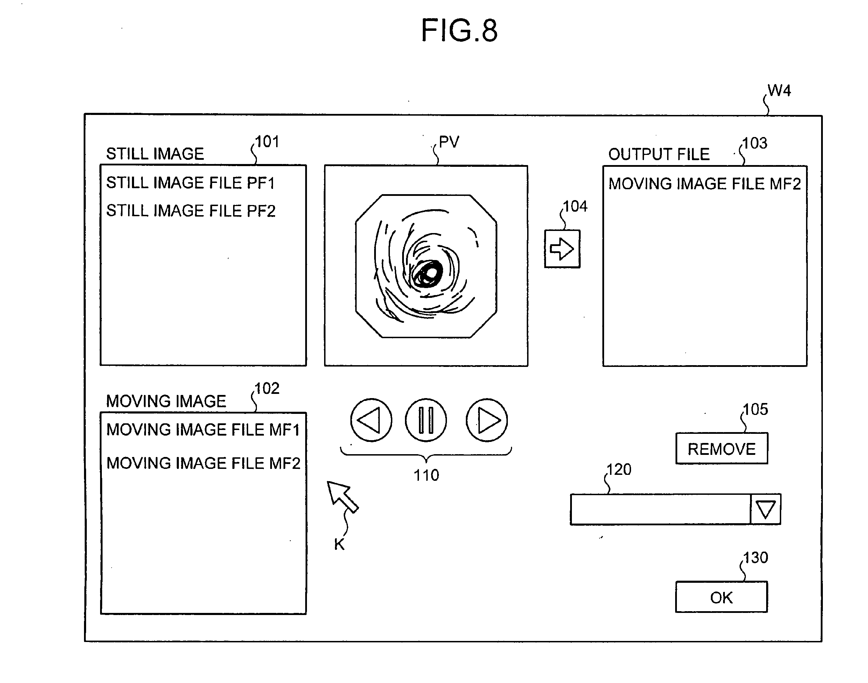

An operation for outputting each image file created by the image file creator 16b to the outside will be described. If the image file created by the image file creator 16b is to be output to the outside, then the user performs an input operation by putting the cursor K and clicking on the image output icon 91 shown in FIG. 4, and displays a predetermined window for performing an image-file output operation or the like. FIG. 8 is a pattern diagram showing a specific example of the window displayed on the display unit 12 for performing the image-file output operation or the like. As shown in FIG. 8, information necessary to output the image files created by the image file creator 16b (that is, the still image file, the normal moving-image file, the combined moving-image file, and the clipped moving-image file) to the outside, icons and the like are formed in a window W4.

Specifically, a still-image file display area 101 for displaying a list of still image files stored in the management folder 15b of the storage unit 15, a moving-image file display area 102 for displaying a list of moving image files stored in the management folder 15b of the storage unit 15, and an output-file display area 103 for displaying a list of files selected to be output to the outside from among those displayed in the still-image file display area 101 or the moving-image file display area 102 are formed in the window W4.

Furthermore, a file designation icon 104 for displaying a desired image file selected and designated from among those in the still-image file display area 101 or the moving-image file display area 102 in the output-file display area 103, and a Remove icon 105 for returning the image file displayed in the output-file display area 103 to its original position (the still-image file display area 101 or the moving-image file display area 102) are formed in the window W4. Moreover, an output-destination selecting area 120 for selecting an output destination of an output-target image filed displayed in the output-file display area 103, and an OK icon 130 for executing a file output processing for outputting the image file to the selected output destination to the output-destination selecting area 120 are formed in the window W4.

Furthermore, a preview area PV for playing still images or moving images included in the image file selected from among those in the still-image file display area 101 or the moving-image file display area 102, and a display-operation icon group 110 for performing operations for displaying the moving images played in the preview area PV are formed in the window W4, and the cursor K is displayed in the window W4.

Still image files PF1 and PF2, for example, are displayed in the still-image file display area 101, and moving image files MF1 and MF2, for example, are displayed in the moving-image file display area 102. In this case, the still image files PF1 and PF2 are still image files created by the image file creator 16b and stored in the management folder 15b of the storage unit 15. The moving image files MF1 and MF2 are normal moving-image files, combined moving-image files or clipped moving-image files created by the image file creator 16b and stored in the management folder 15b of the storage unit 15.

The user performs an operation for selecting a desired still-image file or moving-image file from among those in the still-image file display area 101 or the moving-image file display area 102 using the input unit 11, and then performs an input operation for putting the cursor K on and clicking on the file designation icon 104. In this case, the output processor 16f moves the still-image file or the moving-image file selected and designated by the input operation using the input unit 11 from the management folder 15b into the input/output folder 15c. The still-image file or the moving-image file moved into the input/output folder 15c is displayed in the output-file display area 103.

If the moving image file MF2 in the moving-image file display area 102 is selected and designated, for example, the output processor 16f moves the moving image file MF2 from the management folder 15b into the input/output folder 15c. The moving image file MF2 moved into the input/output folder 15c is displayed as the output-target image file in the output-file display area 103. The output processor 16f sets the moving image file MF2 thus displayed in the output-file display area 103 as the image file to be output to the outside.

Furthermore, if such an input operation for selecting and designating the desired image file is repeatedly performed, the output processor 16f moves the still image file or moving image file from the management folder 15b into the input/output folder 15c whenever the input operation is performed, causes the still image file and the moving image file in the input/output folder 15c to be displayed in the output-file display area 103, and sets the still image file and the moving image file as the output-target image file.

If the Remove icon 105 is selected by an input operation performed by the user using the input unit 11, then the output processor 16f releases all the image files displayed in the output-file display area 103 from the output-target image files, and returns the image files into the original still-image file display area 101 or moving-image file display area 102. In this case, the output processor 16f returns all the image files in the input/output folder 15c into the original management folder 15b.

Thereafter, if the user performs an input operation for selecting the output destination of the image file to be output to the output-destination selecting area 120 using the input unit 11 and performs an input operation for selecting the OK icon 130, then the output processor 16f creates copy files by copying all the still image files and moving image files displayed in the output-file display area 103, that is, all the output-target image files moved into the input/output folder 15c, respectively, and outputs all the obtained copy files to output destinations selected in the output-destination selecting area 120. Examples of the output destinations selected in the output-destination selecting area 120 include a drive of an FD, a CD or a DVD inserted into the information input/output I/F 14 and a peripheral device such as a printer connected through the information input/output I/F 14.

The copy files output to the information input/output I/F 14 by the output processor 16f are stored in the portable recording medium such as the FD, CD or DVD inserted into the information input/output I/F 14, and loaded into the external computer through the portable recording medium. In this manner, the desired still image file or moving image file is output to, for example, the external computer. Specifically, the copy file of the desired still image file or moving image file is output to the external computer or the like through the portable recording medium, and the original file (that is, the still image file or the moving image file), based on which the copy file is created, is held and managed in the management folder 15b of the storage unit 15. In this case, the lock processor 16g performs a data lock processing for prohibiting an update processing and an editing processing on the data in the original file stored in the management folder 15b until the copy file output to the outside is returned to the image display apparatus 4. The data lock processing on the original file performed by the lock processor 16g will be described later.

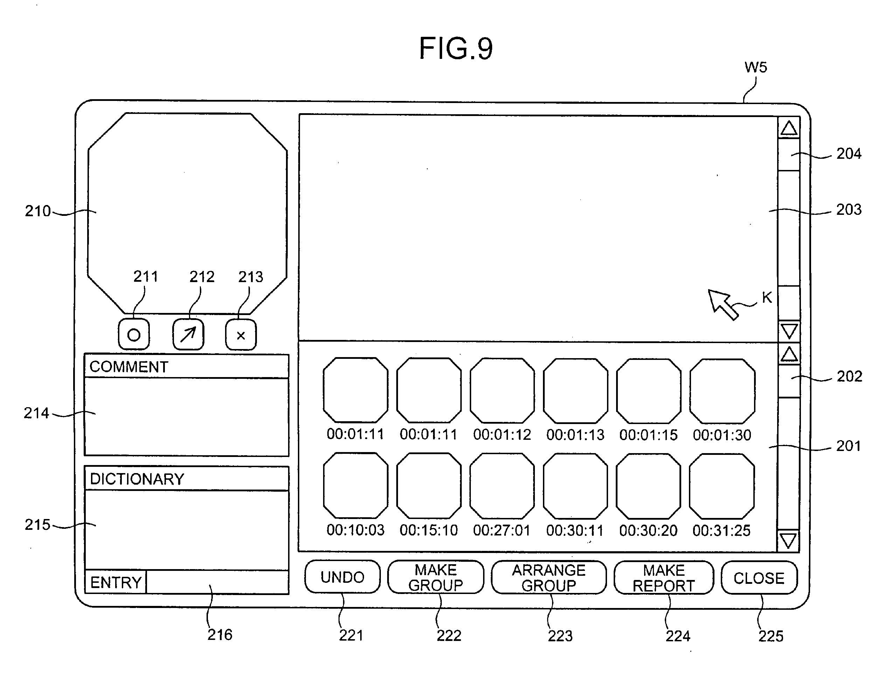

An operation performed by the report creator 16c for creating a report in which the diagnosis result or the like about the subject is recorded, and an operation performed by the group processor 16d for dividing the images recorded in the report into desired groups will next be described. The user observes the images of the subject 1 sequentially displayed in the main-image display area 50, and diagnoses the subject 1. The user performs an input operation for putting the cursor K on and clicking on the report icon 92 shown in FIG. 3 using the input unit 11 to display a predetermined window for creating the report (clinical chart) in which the diagnosis result or the like about the subject 1 is recorded so as to create the report (clinical chart). FIG. 9 is a pattern diagram showing a specific example of the window displayed on the display unit 12 so as to create the report about the subject 1. As shown in FIG. 9, necessary information, icons and the like for creating the report about the subject 1 are formed in a window W5.

Specifically, a thumbnail display area 201 for displaying the thumbnails displayed in the subimage display area 80 in the window W1, a scrollbar 202 for scrolling the thumbnails displayed in the thumbnail display area 201, a grouped-image display area 203 for displaying a result of dividing the thumbnails displayed in the thumbnail display area 201 into desired groups, and a scrollbar 204 for scrolling the grouped images (that is, grouped thumbnails) displayed in the grouped-image display area 203 are formed in the window W5.

Furthermore, an ordinary-image display area 210 for displaying an image corresponding to a desired thumbnail selected from among those in the thumbnail display area 201 or the grouped-image display area 203 (that is, the same image displayed in the main-image display area 50), a marker icon 211 for putting a circular marker or an elliptic marker on the image displayed in the ordinary-image display area 210, a marker icon 212 for putting an arrow marker on the image displayed in the ordinary-image display area 210, and a marker icon 213 for putting a "×" marker on the image displayed in the ordinary-image display area 210 are formed in the window W5. Moreover, a comment creating area 214 for creating comments to be added to the image displayed in the ordinary-image display area 210, a dictionary area 215 for displaying a result of searching a desired word, and an entry area 216 for inputting a letter (e.g., an initial letter) for searching the desired word in the dictionary area 215 are formed in the window W5.

Furthermore, an Undo icon 221 for cancelling various processing performed in the window W5, a grouping icon 222 for dividing the thumbnails displayed in the thumbnail display area 201 into desired groups, a group arrangement icon 223 for changing the arrangement or the like of the grouped images displayed in the grouped-image display area 203, a report creation icon 224 for performing the processing for creating the report on the subject 1, and a Close icon 225 for closing the window W5 are formed in the window W5, and the cursor K is displayed therein.

If the window W5 is opened by selecting the report icon 92 in the window W1, the thumbnails selected from among the images displayed in the main-image display area 50 and displayed in the subimage display area 80 in the window W1 are displayed in the thumbnail display area 201. As shown in FIG. 9, passing time from start of imaging is added to each of the thumbnails in the thumbnail display area 201. To divide the thumbnails displayed in the thumbnail display area 201 into the desired groups, the user selects the thumbnails and performs an input operation for putting the cursor K on and clicking on the grouping icon 222. In this case, the selected thumbnails are grouped and displayed in the grouped-image display area 203.

Thereafter, the group processor 16d stores a result of setting such grouped images in the storage unit 15, thus fulfilling the group setting processing on the thumbnails. The grouped images set by the group processor 16d include one or more thumbnails.

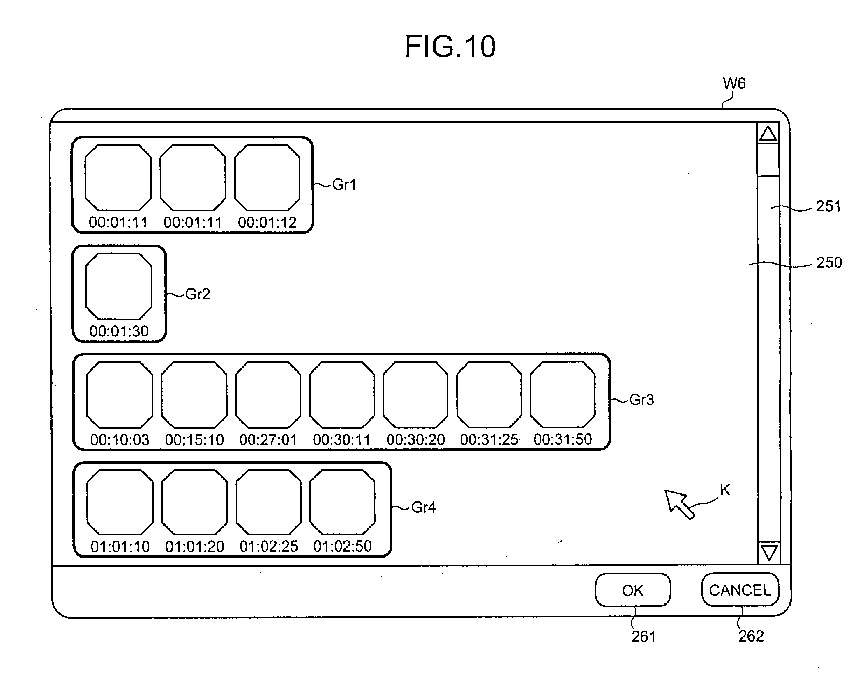

On the other hand, if a window W6 shown in FIG. 10 is opened by an input operation for selecting the group arrangement icon 223 in the window W5, the grouped images in the grouped-image display area 203 are displayed in a grouped area 250 in the window W6. The user performs an arrangement change operation for changing the arrangement or the like of the grouped images displayed in the grouped area 250 using the input unit 11. The group processor 16d changes the arrangement or the like of the grouped images in the grouped area 250 to correspond to the arrangement change operation performed on the grouped images. In this case, the group processor 16d can change the arrangement of the grouped images in order of, for example, Gr1, Gr2, Gr3, and Gr4 shown in FIG. 10 to that in order of Gr1, Gr4, Gr2, and Gr3. Furthermore, if the arrangement change operation for instructing change in the order of thumbnails in a grouped image is performed, the group processor 16d replaces the order of thumbnails in the grouped image to correspond to this arrangement change operation. In this case, the group processor 16d replaces, for example, three thumbnails included in the grouped image Gr1 shown in FIG. 10. The grouped images rearranged by the group processor 16d are displayed in the grouped-image display area 203.

Moreover, a result of the rearrangement processing on the grouped images or thumbnails in the grouped image by the group processor 16d is reflected in recording of the grouped images in the report on the subject. Namely, the report creator 16c records the grouped images in the report on the subject according to the arrangement order of the grouped images rearranged by the rearrangement processing performed by the group processor 16d.

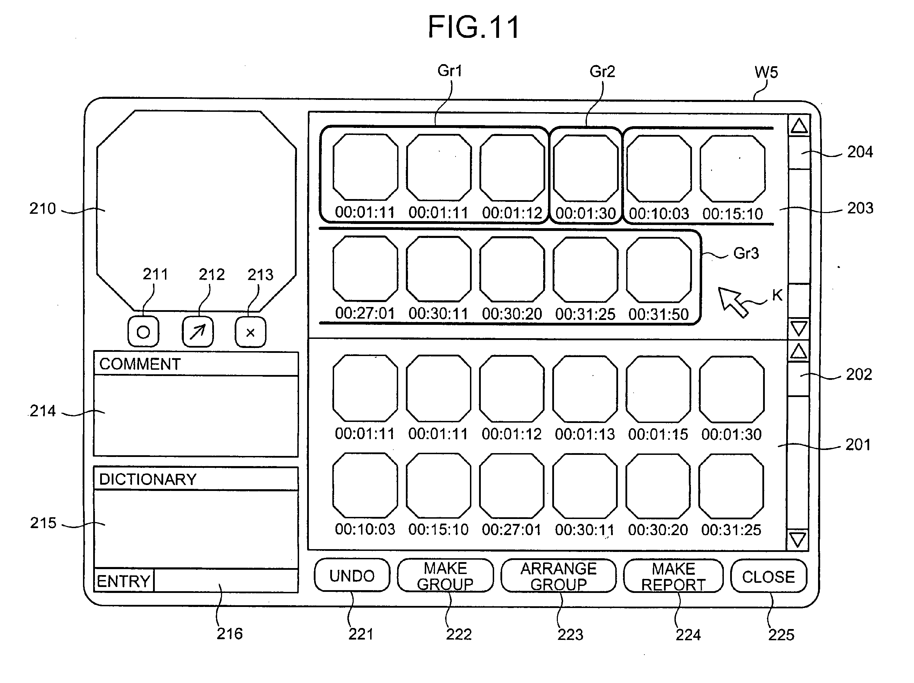

FIG. 11 is a pattern diagram showing an example of the window W5 in a state in which the group processor 16d has set grouped images. As shown in FIG. 11, the grouped images (e.g., grouped images Gr1 to Gr3) set by the group processor 16d are displayed in the grouped-image display area 203. In this case, the report creator 16c functions to put a desired marker such as "o", "→" or "x" for each of the thumbnails included in the grouped images or for the display content of each thumbnail in the thumbnail display area 201.

Specifically, the report creator 16c puts the desired marker such as "o", "→" or "×" for each of the thumbnails included in the grouped images in the grouped-image display area 203 or the image identical in display content to the thumbnail in the thumbnail display area 201. More specifically, the report creator 16c puts the desired marker such as "o", "→" or "x" corresponding to the marker icons 211 to 213, respectively on a desired portion in each of the images displayed in the ordinary-image display area 210. The report creator 16c stores an image editing result for the images, for each of which the desired marker is put, in the storage unit 15, and uses the stored image editing result (that is, the respective images for which the desired markers are put) during creation of the report on the subject.

Moreover, the report creator 16c functions to add desired comments to each of the grouped images in the grouped-image display area 203 or each of the thumbnails in the thumbnail display area 201. Specifically, the report creator 16c makes the comments input to the comment creating area 214 correspond to each grouped image selected from among those in the grouped-image display area 203 or each thumbnail selected from among those in the thumbnail display area 201, and stores the comments made to correspond to each grouped image or thumbnail in the storage unit 15. The report creator 16c uses the comments thus stored during creation of the report about the subject.

Such a report creator 16c includes a dictionary function of facilitating a comment input operation for inputting desired comments into the comment creating area 214. Specifically, the report creator 16c uses dictionary data stored in the storage unit 15 in advance, and searches one or more words including, at the top, a letter input to an entry area. One or more words searched by the report creator 16c are displayed in the form of a list in the dictionary area 215. If a desired word is selected and designated from among search results displayed in the dictionary area 215, the report creator 16c displays the word thus selected and designated in the comment creating area 214. Such a dictionary function of the report creator 16c enables the user to easily perform the comment input operation for inputting the desired comments in the comment creating area 214 using the input unit 11.

If the Undo icon 221 in the window W5 is selected, the report creator 16c returns each image for which the desired marker is put to correspond to one of the marker icons 211 to 213 into an original state in which no marker is put for the image. Moreover, if the Undo icon 221 in the window W5 is selected, the group processor 16d returns the grouped images into an original state in which the images are not grouped.

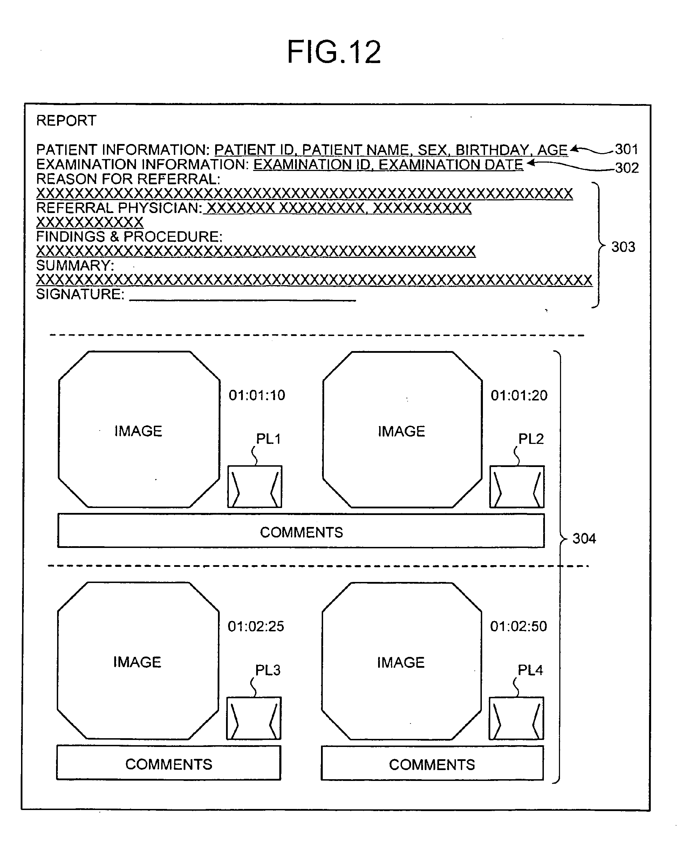

The report creator 16c creates the report on the subject 1 using each of the grouped images, each image identical in display content to each thumbnail in the thumbnail display area 201, each image for which the desired marker is put, and the comments made to correspond to each of the images according to need. FIG. 12 is a pattern diagram showing a specific example of the report created by the report creator 16c. As shown in FIG. 12, the report on the subject 1 is created to include patient information 301 such as the patient name, the sex, the age, the birth date, the patient ID and the like of the subject 1, examination information 302 such as an examination date and an examination ID of a capsule endoscopic examination carried out on the subject 1, diagnosis information 303 such as a diagnosis result for the subject 1, the name of a doctor who diagnosed the subject 1, commends and summary related to the diagnosis result for the subject 1, and examination result information 304 such as the images of the subject 1 and comments. Moreover, a signature space for the doctor who diagnosed the subject 1 is formed in the report on the subject 1.

Specifically, the report creator 16c records the patient information 301 and the examination information 302 in the report about the subject 1 based on the patient information and the examination information included in the examination file for the subject 1 stored in the examination folder 15a of the storage unit 15. If the report creator 16c is instructed to keep the patient information 301 secret by a predetermined input operation using the input unit 11, then the report creator 16c leaves the patient information blank or puts such a marker as "*" in place of the patient information to indicate that the patient information 301 is kept secret.

Moreover, the report creator 16c records information such as the diagnosis result for the subject 1, the doctor who diagnosed the subject 1, and the comments and summary in relation to the diagnosis result of the subject 1 input by the input operation using the input unit 11 in a predetermined field in the diagnosis information 303.

Furthermore, the report creator 16c records the grouped images or the image identical in display content to each thumbnail selected from among those in the thumbnail display area 201, the images for each of which the desired marker is put, the comments and the like made to correspond to each of the images at predetermined positions in the report as the examination result information 304. In this case, the report creator 16c records the images identical in display content with the thumbnails in the grouped images (or the thumbnails themselves) according to the order of arrangement of the grouped images grouped or rearranged by the group processor 16d in the report. Alternatively, the report creator 16c records the images identical in display content with the thumbnails selected from among those in the thumbnail display area 201 (or the thumbnails themselves) in the report in predetermined order of arrangement. If the desired marker is put for each of the images thus recorded, the report creator 16c records the images while the desired marker is put on each of the images. Further, the report creator 16c records comments input into the comment creating area 214 near each of the images thus recorded. The comments are created for each of the grouped images or each of the thumbnails, and recorded near the image made to correspond to the comments by the report creator 16c as shown in FIG. 12. Moreover, the report creator 16c records the passing time of each of the images recorded in the report since start of imaging, and imaged region markers PL1 to PL4 each indicating an imaged region of each image.

In this manner, the report creator 16c creates the report on the subject 1 in which the patient information 301, the examination information 302, the diagnosis information 303, the examination result information 304 and the like are recorded, and stores a report file including the obtained report on the subject 1 in the management folder 15b of the storage unit 15. The report file is output to the printer or the like through, for example, the information input/output I/F by a predetermined input operation using the input unit 11 almost similarly to the image file.

Moreover, if such a report file is to be output to the external computer or the like, then the output processor 16f moves the report file into the input/output folder 15c as the output-target file, creates a copy file of the report file, and outputs the copy file to the information input/output I/F 14 almost similarly to the image data. The copy file output to the information input/output I/F 14 is stored in the portable recording medium such as the FD, CD or DVD inserted into the information input/output I/F 14, and loaded into the external computer through the portable recording medium. In this case, the original file based on which this copy file is created, e.g., the examination file for the subject 1 is held and managed in the management folder 15b of the storage unit 15. The lock processor 16g performs a lock processing for prohibiting an update processing and an editing processing on the original file stored in the management folder 15b until the copy file is returned to the image display apparatus 4 almost similarly to the image file.

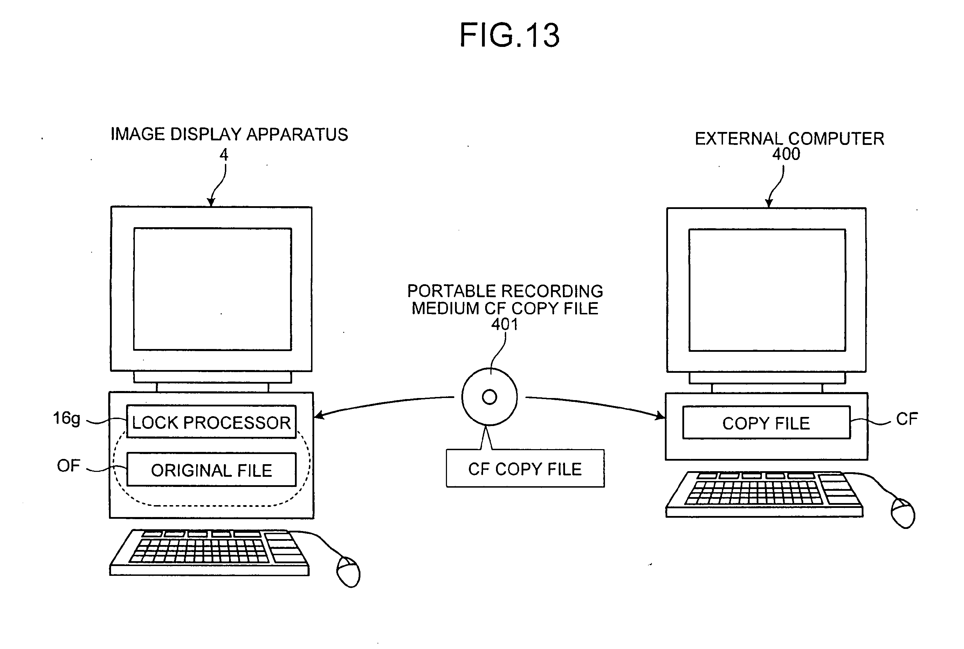

An operation performed by the lock processor 16g for performing the data lock processing on the original file based on which the copy file output to the outside is created will next be described. FIG. 13 is a pattern diagram for explaining the data lock processing performed on the original file by the lock processor 16g. If the image file or the report file in the desired output mode is to be output to the outside, then the output processor 16f creates a copy file obtained by copying the output-target image file or report file, and outputs the obtained copy file to the information input/output I/F 14. In this case, as shown in FIG. 13, a copy file CF is stored in a portable recording medium 401 such as the FD, CD or DVD inserted into the information input/output I/F 14, and loaded into an external computer 400 through the portable recording medium 401. On the other hand, the output processor 16f stores an original file OF (e.g., the image file or the report file), based on which the copy file CF is created, in the management folder 15b of the storage unit 15.

The lock processor 16g functions to acquire a file name of the copy file CF thus output to the outside from the output processor 16f, and to perform the data lock processing on the original file OF identified by the same file name as the acquired file name. Specifically, as shown in FIG. 13, if the copy file CF is output to the outside through the portable recording medium 401, for example, the lock processor 16g performs the data lock processing on the original file OF of this copy file CF until the copy file CF output to the outside is returned to the image display apparatus 4, and turns this original file OF into a data locked state. In this case, even if the user performs an editing operation for rewriting the data (e.g., the still images, the moving image or the report of or on the subject 1) included in the original file OF using the input unit 11, the lock processor 16g prohibits the update processing and the editing processing on the data included in the original file OF to protect the data from being edited or updated.

While the lock processor 16g protects the data in the original file OF, the data in the copy file CF is edited and updated to desired data by the external computer 400. As shown in FIG. 13, the copy file CF updated by the external computer 400 is loaded into the image display apparatus 4 through the portable recording medium 401. Specifically, the control unit 16 reads the copy file CF in the portable recording medium 401 inserted into the information input/output I/F 14 (that is, the copy file CF updated by the external computer 400). In this case, the input processor 16h stores the copy file CF in the input/output folder 15c of the storage unit 15 and acquires the file name of the copy file CF.

The lock processor 16g confirms whether the original file identified by the file name of the copy file acquired by the input processor 16h is stored in the management folder 15b based on the file name of the copy file. Specifically, if the lock processor 16g acquires the original file OF identified by the file name of the copy file CF from within the management folder 15b, then the lock processor 16g releases the data locked state of the original file OF, and permits the editing processing and the update processing on the original file OF. At the same time, the input processor 16h overwrites the data in the copy file CF (that is, the data updated by the external computer 400) on the original file OF, and acquires the original file OF including the data updated by the external computer 400. The input processor 16h stores the original file OF thus acquired in the management folder 15b, and deletes the copy file CF after the original file OF has bee updated. The control unit 16 holds and manages the original file OF in the management folder 15b as a file that can be subjected to the editing processing and the update processing similarly to the other image files or report files.

It has been described that the copy file is input/output between the image display apparatus 4 and the external computer 400 through the portable recording medium 401 detachably attached to the information input/output I/F 14 or the external computer 400. However, the present invention is not limited thereto, and the copy file can be input/output between the image display apparatus 4 and the external computer 400 through a communication line such as a telephone line or the Internet. In this case, the information input/output I/F 14 can be structured to include a communication I/F connectable to the communication line.

Furthermore, it has been described that the lock processor 16g performs the data lock processing on the original file (e.g., original file having the same file name as that of the copy file) identified by the file name of the copy file output to the outside. However, the present invention is not limited thereto, and the lock processor 16g can perform the data lock processing on the original file to which a flag or the like that enables identifying the copy file CF or the original file OF, is added. In this case, the output processor 16f adds flags that enable identifying the copy file CF to be output to the outside and the original file OF to both the copy file CF and the original file OF, respectively, outputs the copy file CF, to which the flag is added, to the outside and stores the original file OF, to which the flag is added, in the management folder 15b. The lock processor 16g performs the data lock processing on the original file OF to which the flag is added, and releases the data locked state of the original file OF if the copy file CF to which the flag is added is acquired.

As described so far, according to the embodiment of the present invention, the image display apparatus is configured to extract a desired partial image group from a series of images of the subject picked up by the capsule endoscope, and to perform a compression processing on images included in the extracted partial image group, thereby generating a partial moving image (normal moving image) that enables this partial image group to be played as a moving image. Furthermore, the image display apparatus is configured, if extracting a plurality of partial image groups, to perform a compression processing on images included in the extracted partial image groups, thereby generating a combined moving image obtained by integrating the partial image groups (that is, a plurality of partial moving images). Moreover, the image display apparatus is configured to extract partial continuous image groups that are continuous image groups arranged between two end images, and to perform a compression processing on images included in the extracted partial continuous image groups, thereby generating a clipped moving image that enables the partial continuous image groups to be played as a moving image. It is, therefore, possible to realize the image display apparatus that can play a partial image group including desired images, e.g., images of the region of interest such as a bleeding site or an affected region, included in all images of a desired subject as a moving image, and that can generate and output moving image data of a small data capacity as compared with a total data capacity of the image group.

By using the image display apparatus according to the present invention, even if the region of interest such as the bleeding site or the affected region is recognized in a plurality of temporally separate images in all the images of the subject, the images in which the region of interest is recognized can be played as a moving image, and the moving image data with a reduced data capacity can be generated and output.

Moreover, the idea that an examination result such as the intra-subject images picked up by the capsule endoscope belongs to the subject, that is, the patient has gradually prevailed, and the examination result such as the moving image data on the subject is often provided to the patient. The image display apparatus according to the present invention is useful for such an instance of providing the examination result to the patient since the moving image data that enables the images of the region of interest included in all images of the subject to be played as a moving image can be output in a state in which the data capacity is reduced.

### INDUSTRIAL APPLICABILITY

As described so far, the image display apparatus according to the present invention can easily output moving image data including images of the region of interest among all the images obtained by sequentially imaging the interior of the subject's digestive canal at time series, and is particularly suitable as the image display apparatus that can reduce the data capacity of the moving image data including the images of the region of interest.

## Claims

1. An image display apparatus (4) for displaying a series of images picked up along time series, **characterised by** comprising:
an input unit (11) adapted to input image designation information for designating a plurality of central images from among the series of images;
an image extracting unit (16e) adapted to extract, from the series of images, a plurality of partial image groups in which the central images designated by the image designation information are arranged at respective centers; and
an image generator (16b) adapted to generate a combined moving image obtained by combining the plurality of partial image groups along time series, based on the plurality of partial image groups extracted by the image extracting unit (16e), wherein
the image generator (16b) is adapted to perform compression processing on the images in the partial image groups and adapted to perform compression processing on the last image in the first partial image group and the first image in the second partial image group.

2. The image display apparatus (4) according to claim 1
, wherein the input unit (11) is further adapted to input end-image designation information for designating two end images from among the series of images,
the image extracting unit (16e) is adapted to extract, from the series of images, a partial continuous image group in which the two end images designated by the end-image designation information are arranged on both ends, respectively, and
the image generator (16b) is adapted to generate a clipped moving image including the partial continuous image group, based on the partial continuous image group extracted by the image extracting unit (16e).

3. The image display apparatus (4) according to claim
2, wherein the input unit (11) is further adapted to input mode designation information for designating an output mode of a moving image generated by the image generator (16b) as one of the partial moving image, the combined moving image, and the clipped moving image, and
the image generator (16b) is adapted to generate the moving image in the output mode designated by the mode designation information.

## Patentansprüche

1. Bildanzeigevorrichtung (4) zum Anzeigen einer Reihe von Bildern, die in Zeitfolge aufgenommen wurden, **dadurch gekennzeichnet, dass** sie aufweist:
eine Eingabeeinheit (11), die dazu eingerichtet ist, Bildkennzeichnungsinformation zum Kennzeichnen einer Mehrzahl von Zentralbildern aus der Reihe von Bildern einzugeben;
eine Bildextraktionseinheit (16e), die dazu eingerichtet ist, aus der Reihe von Bildern eine Mehrzahl von Teilbildgruppen zu extrahieren, in denen die durch die Bildkennzeichnungsinformation gekennzeichnete Zentralbilder an jeweiligen Zentren angeordnet sind; und
einen Bildgenerator (16b), der dazu eingerichtet ist, ein kombiniertes bewegtes Bild zu erzeugen, das erlangt wurde durch Kombinieren der Mehrzahl von Teilbildgruppen in Zeitfolge, basierend auf der durch die Bildextraktionseinheit (16e) extrahierten Mehrzahl von Teilbildgruppen, wobei
der Bildgenerator (16b) dazu eingerichtet ist, Kompressionsverarbeitung an den Bildern in den Teilbildgruppen auszuführen, und dazu eingerichtet ist, Kompressionsverarbeitung an dem letzten Bild in der ersten Teilbildgruppe und dem ersten Bild in der zweiten Teilbildgruppe auszuführen.

2. Bildanzeigevorrichtung (4) nach Anspruch 1, bei der die Bildeingabeeinheit (11) ferner dazu eingerichtet ist, Endbildkennzeichnungsinformation zum Kennzeichnen von zwei Endbildern aus der Reihe von Bildern einzugeben,
die Bildextraktionseinheit (16e) dazu eingerichtet ist, aus der Reihe von Bildern eine teilweise kontinuierliche Bildgruppe zu extrahieren, in der die durch die Endbildkennzeichnungsinformation gekennzeichneten zwei Endbilder jeweils an beiden Enden angeordnet sind, und
der Bildgenerator (16b) dazu eingerichtet ist, ein geclipptes bewegtes Bild, das die teilweise kontinuierliche Bildgruppe enthält, basierend auf der durch die Bildextraktionseinheit (16e) extrahierte teilweise kontinuierliche Bildgruppe zu erzeugen.

3. Bildanzeigevorrichtung (4) nach Anspruch 2, bei der die Eingabeeinheit (11) ferner dazu eingerichtet ist, Modus-Kennzeichnungsinformation zum Kennzeichnen eines Ausgabemodus eines durch den Bildgenerator (16b) erzeugten bewegten Bildes als eines des teilweise bewegten Bildes, des kombinierten bewegten Bildes und des geclippten bewegten Bildes zu kennzeichnen, und
der Bildgenerator (16b) dazu eingerichtet ist, das bewegte Bild in dem durch die Modus-Kennzeichnungsinformation gekennzeichneten Ausgabemodus zu erzeugen.

## Revendications

1. Appareil (4) d'affichage d'images destiné à afficher une série d'images capturées en série temporelle, **caractérisé en ce qu'**il comprend :
une unité d'entrée (11) adaptée pour délivrer en entrée des informations de désignation d'images destinées à désigner une pluralité d'images centrales parmi la série d'images ;
une unité (16e) d'extraction d'images adaptée pour extraire, à partir de la série d'images, une pluralité de groupes d'images partielles dans lesquels les images centrales désignées par les informations de désignation d'images sont agencées au niveau des centres respectifs ; et
un générateur (16b) d'images adapté pour générer une image mobile combinée obtenue en combinant la pluralité de groupes d'images partielles dans la série temporelle, sur la base de la pluralité de groupes d'images partielles extraites par l'unité (16e) d'extraction d'images,
dans lequel
le générateur (16b) d'images est adapté pour réaliser un traitement de compression sur les images dans le groupe d'images partielles et adapté pour réaliser un traitement de compression sur la dernière image dans le premier groupe d'images partielles et la première image dans le deuxième groupe d'images partielles.

2. Appareil (4) d'affichage d'images selon la revendication 1, dans lequel l'unité d'entrée (11) est en outre adaptée pour délivrer en entrée des informations de désignation d'images d'extrémité destinées à désigner deux images d'extrémité à partir de la série d'images,
l'unité (16e) d'extraction d'images est adaptée pour extraire, à partir de la série d'images, un groupe d'images continues partielles dans lequel les deux images d'extrémité désignées par les informations de désignation d'images d'extrémité sont agencées aux deux extrémités, respectivement, et
le générateur (16b) d'images est adapté pour générer une image mobile coupée incluant le groupe d'images continues partielles, sur la base du groupe d'images continues partielles extraites par l'unité (16e) d'extraction d'images.

3. Appareil (4) d'affichage d'images selon la revendication 2, dans lequel l'unité d'entrée (11) est en outre adaptée pour délivrer en entrée des informations de désignation de mode destinées à désigner un mode de sortie d'une image mobile générée par le générateur (16b) d'images en tant que l'une parmi l'image mobile partielle, l'image mobile combinée, et l'image mobile coupée, et
le générateur (16b) d'images est adapté pour générer l'image mobile dans le mode de sortie désigné par les informations de désignation de mode.
